**Europäisches Patentamt**

**European Patent Office**

**Office européen des brevets**

(19)

(11) Veröffentlichungsnummer: **0 013 268**
**B1**

(12) **EUROPÄISCHE PATENTSCHRIFT**

(45) Veröffentlichungstag der Patentschrift: **15.12.82**

(51) Int. Cl.³: **A 61 L 2/02** //G01T1/11

(21) Anmeldenummer: **79890067.6**

(22) Anmeldetag: **19.12.79**

(54) Verfahren zur Sterilisation von Thermolumineszenzdosimetern.

(30) Priorität: **22.12.78 AT 9227/78**

(43) Veröffentlichungstag der Anmeldung:
**09.07.80 Patentblatt 80/14**

(45) Bekanntmachung des Hinweises auf die Patenterteilung:
**15.12.82 Patentblatt 82/50**

(84) Benannte Vertragsstaaten:
**CH DE**

(56) Entgegenhaltungen:
CH - A - 564 947
FR - A - 2 240 742

HEALTH PHYSICS, Band 16, Nr. 6, Juni 1969,
Pergamon Press, New York, US,
J. KASTNER: "Ultrasonic excitation of
Thermoluminescent Lithium Fluoride" * Seiten
803,804 *

(73) Patentinhaber: **Österreichisches**
**Forschungszentrum Seibersdorf Gesellschaft**
**m.b.H.**
**Lenaugasse 10**
**A-1082 Wien (AT)**

(73) Patentinhaber: **Stadt Wien**
**Neues Rathaus**
**A-1141 Wien (AT)**

(72) Erfinder: **Merkel, Karl Dr. med.**
**Hirschengasse 6**
**A-1060 Wien (AT)**
Erfinder: **Ryvarden, Gunnar, Dr.med.**
**Michaelerstrasse 23, St. 18**
**A-1180 Wien (AT)**

Courier Press, Leamington Spa, England.

# Verfahren zur Sterilisation von Thermolumineszenzdosimetern

Die Erfindung bezieht sich auf ein Verfahren zur Sterilisation von Thermolumineszenzdosimetern, insbesondere von Lithiumfluoriddosimetern, unter Aufrechterhaltung der gespeicherten Information über die im Dosimeter absorbierte Strahlendosis, in dem das Dosimeter in einer Flüssigkeit mit Ultraschall mit einer Frequenz im Bereich von 40 kHz beschallt wird.

Durch die verschiedensten Heil- und Diagnoseverfahren, bei welchen Strahlen eingesetzt werden, wird der behandelnde Arzt und das Hilfspersonal einer zusätzlichen Strahlungsbelastung ausgesetzt. Aus der Literatur und aus Verordnungen der zuständigen Behörden sind höchstzulässige Grenzen für die Vierteljahres-, die Jahres- und die Lebensalterdosis bekannt. Diese Toleranzgrenzen sind so festgelegt, daß eine Schädigung des der Strahlung ausgesetzten Personenkreises vermieden wird. Voraussetzung für die Einhaltung dieser Toleranzen ist allerdings, daß diese Personen Dosimeter tragen, welche über die entsprechende Empfindlichkeit verfügen und von der betreffenden Person während der Zeit einer möglichen Strahlungsbelastung getragen werden. Das sogenannte Thermolumineszenzdosimeter erfüllt die gestellten Anforderungen. Dosimeter dieser Bauart weisen beispielsweise Lithiumfluorid-Kristalle auf, die beispielsweise auf einer kleinen Lochkarte befestigte sind, wodurch eine vollautomatische Auswertung erleichtert ist. Derartige Thermolumineszenzdosimeter erlauben selbst bei der Routineanwendung noch eine Dosis von 3 mrem genau zu erfassen. Die Auswertung der gespeicherten Daten ist relativ einfach. Die mit Strahlung beaufschlagten Dosimeter werden erhitzt, wobei eine Lichtemission auftritt. Der physikalische Ablauf ist wie folgt: Die im Detektorkristall durch Strahlung freigesetzten Elektronen werden in Fehlstellen des Kristallgitter eingefangen und werden in dieses als Information über die vom Detektor absorbierte Dosis gespeichert. Führt man dem Detektor jedoch, z.B. durch Erwärmung, zusätzliche Energie zu, so werden die Elektronen aus den Fehlstellen des Kristallgitters freigesetzt und geben die hiebei freiwerdende Energie in Form von sichtbarem Lumineszenzlicht ab. Die Intensität dieser Lumineszenz stellt daher ein Maß für die im Kristall absorbierte Strahlendosis dar. Die für die in vielen Bereichen der medizinischen Anwendung dieser Thermolumineszenzdosimeter notwendige Sterilisation von Thermolumineszenzdosimetern, z.B. Fingerringdosimetern kann daher nicht mit der üblicherweise durchgeführten Wasserdampfsterilisation bei 134°C erfolgen, da die im Dosimeter gespeicherten Informationen hiedurch teilweise gelöscht werden. Das für die Sterilisation übliche rein chemische Desinfektionsverfahren, z.B. mit gasförmigen Desinfektionsmitteln wie Äthylenoxyd, ist prinzipiell geeignet, bedingt jedoch einen sehr hohen Zeitaufwand, so daß die Gefahr besteht, daß die Benützung des Dosimeters aus Zeitgründen entweder unterbleibt oder mehrere Dosimeter verwendet werden, wobei die Gefahr einer nicht vollständigen Erfassung der Strahlungsbelastung durch Unachtsamkeit des Benützers bzw. auch die Gefahr von Verwechslungen sehr groß ist. Bei der Verwendung mehrerer Dosimeter akkumulieren auch die durch die Auswertung bedingten Ungenauigkeiten.

Ein anderes Verfahren zur Sterilisation, das bereits auf dem medizinischen Sektor eingeführt ist, beruht auf der Wirkung von Ultraschall. Mit Ultraschall werden jene, mechanischen Schwingungen in festen, flüssigen oder gasförmigen Körpern bezeichnet, deren Frequenz jenseits der Hörgrenze liegt. Es existiert ein eigener Zweig der Chemie, die sogenannte Sonochemie, die sich mit der Umsetzung von chemischen Stoffen unter Einwirkung von Ultraschall beschäftigt. So ist beispielsweise bekannt, daß Tetrachlorkohlenstoff unter Einwirkung von Ultraschall unter Chlorbildung zersetzt wird, Acrylnitril polymerisiert, bei Aluminium kann der Oxidfilm zerstört werden und dgl. Zur Erklärung dieser Phänomene wird angeführt, daß sich in den Flüssigkeiten sogenannte Kavitationsblasen bilden, bei deren Kollaps kurzzeitig Temperaturen zwischen 400 und 2.400°C auftreten. Die Desinfektionswirkung von Ultraschall wird einerseits durch die mechanische Zerstörung der Mikroorganismen und anderseits, z.B. durch Entstehen von Desinfektionsmittel in der Flüssigkeit — Wassersuperoxid bei Wasser — erklärt.

So wird z.B. in der FR—A—2 240 742 ein Verfahren beschrieben, bei welchem unter Anwendung von Ultraschall die Entkeimung von Kontaktlinsen und Ähnlichem mit und ohne Temperatureinwirkung, erfolgt.

In anderen Verfahren wird ganz allgemein ohne Hinweis auf bestimmte zu sterilisierende Gegenstände die Kombination von Ultraschallsterilisation mit üblichen chemischen Sterilisationsverfahren, die bei relativ niederen Temperaturen anwendbar sind, beschrieben. So wird z.B. in CH—A—564 947 die Kombination von Ultraschall mit Entkeimungskompositionen wie z.B. Glutaraldehyd und nichtionische Tenside, beschrieben.

Das erfindungsgemäße Verfahren zur Sterilisation von Thermolumineszenzdosimetern besteht im wesentlichen darin, daß das Dosimeter in eine mit Ultraschall der bezeichneten Frequenz beaufschlagte Flüssigkeit getaucht wird. Für den Fachmann, dem die mechano-chemischen und energetischen Wirkungen des auf eine Flüssigkeit einwirkenden Ultraschalls bekannt sind, wurde völlig überraschend festgestellt, daß trotz der Energiezufuhr in das Dosimeter in Form von Ultraschall die gespeicherte Information über die absorbierte Strahlendosis praktisch nicht verändert wird, obwohl durch die Einwirkung des Ultraschalls eine einwandfreie Sterilisation des Dosimeters innerhalb kurzer Zeit bewirkt wird. Eine besonders vorteilhafte Ausführung des erfindungsgemäßen Verfahrens, welches sich in einer wesentlichen Verkürzung des für die einwandfreie Sterilisation erforderlichen Zeit auswirkt, besteht darin, daß in der Flüssigkeit, auf die der Ultraschall einwirkt, ein Desinfektionsmittel enthalten ist. Die Überprüfung der Sterilität der Dosimeter erfolgte jeweils mittels Testsporenlösung.

Im folgenden wird die Erfindung anhand von Beispielen näher erläutert.

### Beispiel 1

Zehn Lithiumfluoridkristalldosimeter wurden mit einer Dosis von je entsprechend 200 mR bestrahlt. Die so bestrahlten Dosimeter wurden 2 Minuten auf 97°C erhitzt, so daß etwa anhaftende Flüssigkeitsfilme verdampfen und damit die nachfolgende fotometrische Auswertung nicht stören. Die so thermisch vorbehandelten Dosimeter wurden sodann in einem Fotometer der Firma Harshaw (TL-Detector-Dosimeter Identifier Modell 2271) ausgewertet, dabei werden die Dosimeter in 10 Sekunden auf 300°C erhitzt und es wird die freigesetzte Lichtmenge bestimmt. Als arithmetisches Mittel hat sich ein Wert von 198 mR ergeben.

### Beispiel 2

Die im Beispiel 1 angeführten 10 Dosimeter wurden in einer 10-%igen Glutaraldehydlösung getaucht, worauf zwei Minuten lang das Entweichen der Luftblasen abgewartet wurde und worauf 35 Minuten lang Ultraschall mit einer Frequenz von 40 kHz beaufschlagt wurde. Die Dosimeter wurden getrocknet und nachfolgend mit 200 mR bestrahlt. Die Auswertung, welche analog Beispiel 1 durchgeführt wurde, hat einen Mittelwert von 199 mR ergeben.

### Beispiel 3

Es wurden analog den Beispielen 1 und 2 vorgegangen, wobei die Dosimeter zuerst bestrahlt und dann mit Ultraschall sterilisiert wurden. Als arithmetisches Mittel wurde 200 mR gefunden.

### Beispiel 4

Auf fünf Fingerring-Dosimeter wurden mittels [60]Costrahler je eine Dosis von 500 mR aufgebracht. Drei dieser bestrahlten Dosimeter wurden an 20 Werktagen dreimal täglich mit Ultraschall sterilisiert, — das sind insgesamt 60 Sterilisationen für jedes der drei erwähnten Fingerring-Dosimeter. Die restlichen zwei bestrahlten Dosimeter blieben zur Kontrolle unbehandelt, d.h. unbeschallt. Nach Abschluß der Sterilisationsperiode wurde überprüft, ob die eingespeicherte Information der Fingerring-Dosimeter durch die Ultraschallsterilisation eine Änderung erfahren hatte. Die Auswertung der Dosimeter ergab für die mit Ultraschall desinfizierten Dosimeter $500 \pm 31$ mR und für die Kontrolldosimeter $500 \pm 33$ mR. Aus diesem Vergleich ergibt sich, daß unter praxisbezogenen Versuchsbedingungen auch durch gehäufte Bestrahlung die gespeicherte Information der Fingerring-Dosimeter nicht signifikant verändert wird.

Den obengenannten Beispielen ist in eindeutiger Weise zu entnehmen, daß die Ultraschallbehandlung weder vor noch nach der Bestrahlung einen störenden Einfluß auf die Registrierung der Strahlendosis besitzt.

### Patentansprüche

1. Verfahren zur Sterilisation von Thermolumineszenzdosimetern, insbesondere von Lithiumfluoriddosimetern, unter Aufrechterhaltung der gespeicherten Information über die im Dosimeter absorbierte Strahlendosis, dadurch gekennzeichnet, daß das Dosimeter in eine Flüssigkeit eingetaucht und darin mit Ultraschall mit einer Frequenz im Bereich von 40 kHz beschallt wird.

2. Verfahren nach Anspruch 1, dadurch gekennzeichnet, daß eine Desinfektionsmittel enthaltende Flüsssigkeit Verwendung findet.

3. Verfahren nach einem der Ansprüche 1 oder 2, dadurch gekennzeichnet, daß als Flüssigkeit Wasser Verwendung findet.

### Revendications

1. Procédé de stérilisation de dosimètres à thermoluminescence, en particulier de dosimètres au fluorure de lithium, conservant intacte l'information sur la dose de rayons absorbée dans le dosimètre, caractérisé en ce que le dosimètre est plongé dans un liquide où il est exposé à l'action des ultrasons à une fréquence de l'ordre de 40 kHz.

2. Procédé selon la revendication N° 1, caractérisé en ce que le liquide utilisé contient un désinfectant.

3. Procédé selon la revendication N° 1 ou 2, caractérisé en ce que le liquide utilisé est de l'eau.

### Claims

1. A process to sterilize thermoluminescene dosimeters, in particular lithium fluoride dosimeters, where the stored information on the radiation dose absorbed by the dosimeter is retained, characterized by the fact that the dosimeter is immersed in a liquid and exposed to ultrasonic waves of the 40 kHz frequency range.

2. A process as claimed in Claim 1, characterized by the fact that the liquid used contains a disinfectant.

3. A process as claimed in Claim 1 or 2, characterized by the fact that the liquid used is water.